# EUROPEAN PATENT APPLICATION

(11) **EP 2 515 111 A1**
(43) Date of publication of application: **24.10.2012**
(21) Application number: 11003275.2
(22) Date of filing: 19.04.2011
(51) Int. Cl.: G01N 33/543, G01N 33/554, G01N 33/569

(54) **Analytical method to investigate binding of analyte ligands to cells**

(71) Applicant: SAW instruments GmbH, 53177 Bonn (DE)
(72) Inventor: Gronewold, Thomas, 53177 Bonn (DE); Perpeet, Markus, 53177 Bonn (DE)
(74) Representative: Nordmeyer, Philipp Werner

(57) **Abstract**

The present invention relates to improved methods to detect and/or investigate the interaction between an analyte ligand and a cell or cell-like particle. Such methods use, in a particular way, a particular mass-sensitive chemical sensor comprising a surface acoustic wave device, to detect a particular property of an acoustic wave stimulated on the surface of such detector. The methods of the present invention are flexible and easy to perform, and can be used for example, to detect and investigate antibody interaction to extra-cellular domains of membrane proteins presented on intact mammalian cells.

## Description

The present invention relates to improved methods to detect and/or investigate the interaction between an analyte ligand and a cell or cell-like particle. Such methods use, in a particular way, a particular mass-sensitive chemical sensor comprising a surface acoustic wave device, to detect a particular property of an acoustic wave stimulated on the surface of such detector. The methods of the present invention are flexible and easy to perform, and can be used, for example, to detect and investigate antibody interaction to extra-cellular domains of membrane proteins presented on intact mammalian cells.

Current commonly used label-free biosensing techniques such as SPR cannot use whole cells, and is not able to work reliably except with soluble proteins in clean buffer. This prevents the ability to investigate ligand binding to an important class of proteins - membrane proteins - and in particular to investigate ligand binding to whole cells.

Cell-receptors and other extra-cellular domains of membrane proteins are an important class of drug targets, and are the focus of significant research efforts in many laboratories around the world. The detection and other investigation of the interaction between such proteins (or other cell-surface displayed components such as carbohydrates and lipids) and their respective ligands that are present external to the cell is an important tool to a better understanding of such surface-bound molecules, and potentially could lead to the discovery, understanding and/or development of different or improved ligands that interact with such surface-bound molecules as potential therapeutic candidates. For example, a better understanding of the specificity or kinetics of the interaction between an antibody ligand (such as trastuzumab) and its membrane bound target (HER2) that is expressed on certain breast cancer cells could lead to the development of improved treatments for breast cancer. Similarly, a better understanding of the interaction between a non-antibody therapeutic such as erythropoetin and its cell-surface receptor could lead to the development of improved treatments for kidney disease or cancer.

In other applications, a better understanding of those interactions formed between intact mammalian cells and other ligands - such as viral particles, invasive bacteria, other eukaryotic cells such as immunomodulatory cells or eukaryotic parasites, carbohydrates, glycoproteins cholesterol or fat particles, nucleic acids and organic molecules - would greatly aid the understanding of the biology of such mammalian cells and of the consequence of such interactions, and could lead to important scientifically or medically relevant discoveries.

The applications described above, and many others too, assume that the interaction between a cell and a ligand that can be detected or investigated in an isolated system in the laboratory is qualitatively and quantitatively the same interaction as that which occurs in the cell's native environment, such as the interaction between trastuzumab and HER2 actually within the breast tumour of a woman suffering from breast cancer, and that what is detected and/or investigated is not a consequence of, or materially affected by, the conduct of such investigations in an isolated and artificial laboratory setting. Accordingly, there is great need to detect such interactions using a system that keeps the cells in as close to their native state as possible. For example, it is desired that the cell to be investigated remains intact (i.e. it was not lysed or fragmented), alive and/or not treated with chemicals (such as formaldehyde) or other harsh conditions (such as UV light) that might significantly affect the biochemical or morphological characteristics of such cell. In particular, prior-art detection methods that required such treatments to the cell before it can be investigated, may cause significant changes to the cell-surface bound protein that forms one part of the interaction - for example denaturation, removal, or cross-linking of such protein - and these changes may cause such protein to no longer interact with its respective ligand in a manner that occurs in nature, or at all. Data generated under such circumstances, and conclusions made on such interactions, will therefore need to be interpreted with caution.

The need for improved techniques for detection or investigation of interaction between an analyte ligand and a particle is not limited to particles that are cells. The study of such interactions with cell-like particles is also of great interest in various fields of research. For example, a method for the detection of interactions between liposomes (an example of a "cell-like particle") and carbohydrates, lipids, drugs or even cells is also desired. Ligand interactions with other cell-like particles are also desired. Examples of other such cell-like particles include nano- or microparticles (typically ranging in size from about 10 nm to 200um, such as between about 1 to 10um, or between about 5 and 20um), such as those made from lipid layers, polymers or gels, and lipoproteins.

Accordingly, the present invention aims to provide improved or alternative methods for detecting and/or investigating an interaction between an analyte ligand and a cell or a cell-like particle; in particular, to provide methods that enable the detection and/or investigation of such an interaction to cells that have not been inactivated, but rather are intact and/or are native cells, such as whole eukaryotic cells.

WO2010/106331 describes a method using a mass-sensitive chemical sensor having cells adhered to a sensing surface thereof to detect an interaction between an analyte ligand and the adhered cells. The mass-sensitive chemical sensors described therein are bulk-wave quartz crystal microbalances (QCM) and acoustic wave devices, which are used therein to detect a mass-change upon a change in resonance frequency of the device. Of significance however, is that the method described in WO2010/106331 is limited to inactive cells, described therein as where the "normal biochemistry of the cells is substantially arrested, such that the cells are substantially no longer capable of growth, division, movement and/or morphological changes" [emphasis added]. Indeed, cell inactivity is essential for the claimed methods, as increasing the rigidity of the cell layer improves the sensitivity of the sensor by extending the sensing range of the acoustic sensor in the cell layer, particularly when eukaryotic cells (with sizes in the 10um range) are used, as otherwise the interaction "may not be completely sensed by the piezoelectric sensor". Such "beneficial effects" of a rigid cell layer is gained in the method described therein by harsh treatments to a pre-formed and pre-incubated cell layer on the sensing surface using chemical fixation and/or crosslinking methods. For example the use of organic solvents and crosslinking reagents, such as formaldehyde, to fix the cells are described. Organic solvent based methods typically work by removing lipids and water from the cells and precipitate cellular proteins, whereas cross-linking reagents form intermolecular bridges between surface components of cells. In each case, substantial changes to the cell, cell-surface and/or to the protein target will occur. Indeed, formaldehyde is commonly known as a preservative or fixative of tissues for histological or embalming procedures, and it preserves and fixes tissue or cells by reversibly cross-linking primary amino groups in proteins with other nearby nitrogen atoms in protein or DNA through a -CH₂- linkage. It is the ability of formaldehyde to "fix" tissue that produces a tell-tale firmness of flesh in an embalmed body, and hence it is this ability that is presumed to cause the rigidity of cells required for the method described in W02010/106331.

Other disadvantages of the method described in WO2010/106331 arise because of a step requiring the cells are first grown on the sensor surface before their subsequent fixation. Accordingly: (i) preparation of a device ready for use needs substantial time and steps as described therein; (ii) the morphology and biochemistry of the cells may be affected by surface-growth (even before any changes brought about inactivation, e.g. by chemical fixation); (iii) the types of cells that can be studied will be greatly limited to those amenable to be cultured on a sensor surface; and (iv) the fixed/cross-linked cells cannot easily be removed from the sensor surface, and hence it cannot easily be regenerated or reused - not only increasing the cost per-experiment, but limiting the ability to make certain control experiments.

Surface acoustic wave (SAW) sensors have been used in a variety of applications as biosensors and in the bioanalytical field. Recent comprehensive reviews of these applications and various improvements to SAW-sensor technology have been written, and include reviews from Gronewold (2007; Anal Chim Acta, 603:119-128) and Laenge et al (2008; Anal Bioanal Chem, 391:1509-1519). Particular prior-art applications and improvements of SAW sensors include the following representative disclosures.

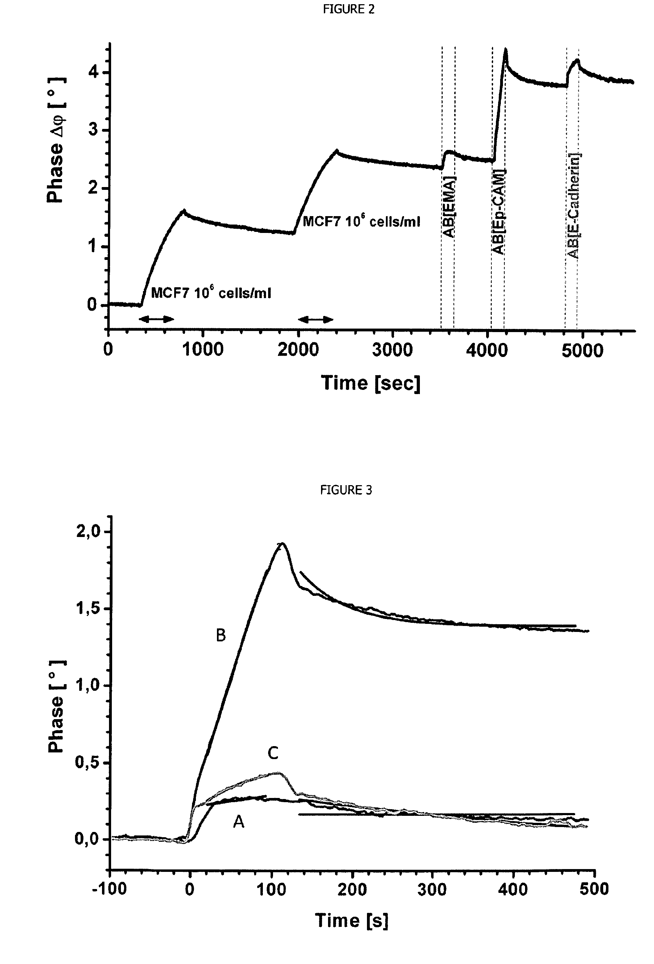

Saitakisa et al (2010; Biosen Bioelec, 25:1688-1693) and WO2009/037660 describe the use of a Love-wave SAW-based sensor to detect and analyse the interaction of cell membrane bound molecules to a ligand. In such method, the ligand is attached to the sensor surface (see Fig 2 of WO2009/037660), and the interaction to cells are then bound to said immobilised ligand, as the device sensed those binding events that occurred within the relatively short distance (50nm) from the device surface.

SAW-based sensors have been used to detect the changes in growth or morphology of various kinds of cells. In particular, recently Gammoudi and co-workers (2010; Biosen Biochem, 26:1723-1726, and 2011; Sens Lett, 9:816-819) describe the use of a Love-wave bacterial-based sensor for the detection of heavy metal toxicity in liquid medium. The SAW sensor (described as an "acoustic delay-line") is inserted in an oscillation loop in order to record the resonance frequency in real-time. E.coli bacteria as biosensors are fixed into the sensitive surface of the sensor coated with a polyelectrolyte multilayer. Real time resonance-frequency perturbations in response to various concentrations of cadmium and mercury are investigated, which perturbations are attributed to changes in the viscoelastic properties of the bacteria related to modifications in the metabolism of the bacteria.

Other forms of polymer coatings have been used to enhance the sensitivity, loading capacity or other properties of SAW sensor surfaces, and include hydrogels as described in US7473551. Such sensors with a hydrogel located on the surface are further disclosed to include within the hydrogel an immobilized molecular recognition component capable of detecting an analyte. Amongst numerous possible molecular recognition components are disclosed prokaryotic and eukaryotic cells.

Howe and Harding (2000; Biosen Bioelec, 15:641-649) describe a novel protocol for the detection and quantification of bacteria using a SAW biosensor. Such protocol included first coating the bacteria to be detected on the sensor surface prior to addition of an antibody to detect such bacteria. The Love-wave sensors so loaded with bacteria were connected with a broadband amplifier between the transmit- and receive- inter-digital transducers (IDTs) to detect mass-loading at the surface of the sensor by a decrease in the characteristic oscillation frequency of the device.

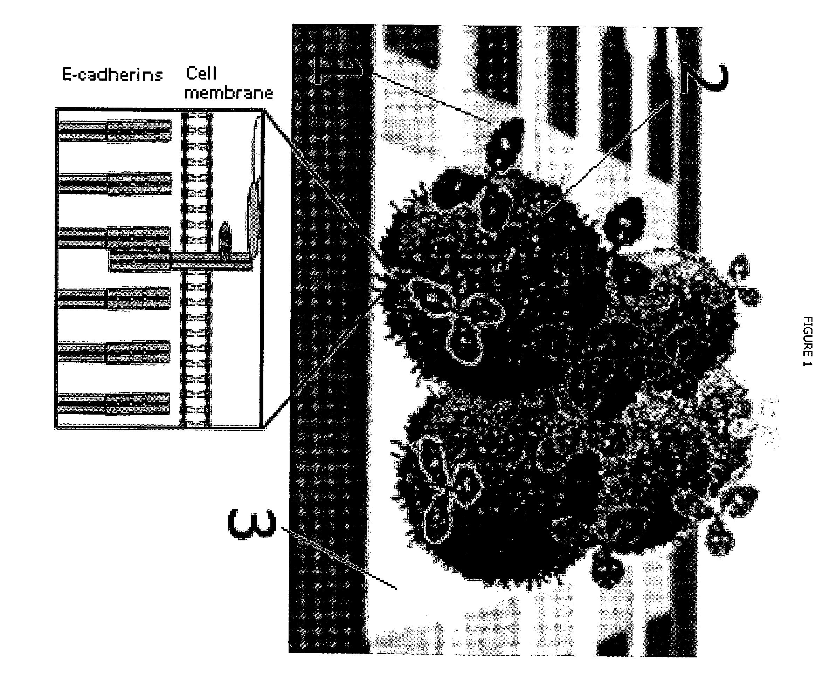

Andrae and cowokers (2008; Langmuir 24:9148-9153) described a surface acoustic wave biosensor as a tool to study the interaction of antimicrobial peptides with phospholipid and lipopolysaccharide model membranes. They developed a technique that absorbed "lipid aggregates/liposomes" onto the surface of a Love-wave sensor, yet resulted not in cell-like particles but in a stable lipid bilayer that covered the chip surface homogenously (see Figure 1 and atomic force microscopy images and scale in Figure 7 therein). These homogeneous bilayers absorbed on the sensor allowed the investigation of binding and intercalation of membrane-active peptides. The peptide-membrane interaction resulted in a positive phase shift and an increase in amplitude, indicating a mass increase along with a loss in viscosity. This suggested that the bilayer became more rigid upon interaction with the membrane-active peptide.

Despite all these prior art applications and developments, there is a need for improved and/or alternative methods to detect and/or investigate the interaction between an analyte ligand and a cell or a cell-like particle. The inventors made the surprising invention, as claimed, defined or otherwise described herein, that through the use in a particular way of a specific type of acoustic wave-based mass-sensitive chemical detector, and by detecting a change in a particular property of an acoustic wave stimulated on such detector, they could detect, investigate and even quantitate the interaction between an analyte ligand and a cell or a cell-like particle, in particular with cells that have not been inactivated, but rather are intact and/or are native cells, such as whole eukaryotic cells.

### SUMMARY

In a first aspect, the invention relates to **a method of detecting and/or investigating an interaction** between an analyte ligand and a cell or a cell-like particle, said method comprising the steps of:
- Providing a mass-sensitive chemical sensor having said cells or cell-like particles adhered to a sensing surface thereof, wherein the sensor is adapted to allow liquid to be introduced into the vicinity of the sensing surface; and
- Introducing a liquid comprising the analyte ligand into the vicinity of the sensing surface so as to allow interaction between analyte ligand and cells or cell-like particles; and
- Where an interaction is present, detecting and/or investigating the interaction by means of the change in mass at the sensing surface;
   wherein, said mass-sensitive chemical sensor comprises a surface acoustic wave device adapted to: (a) stimulate a shear-horizontal surface acoustic wave (SH-SAW) upon the application of an excitation signal; (b) propagate said wave through a guiding-layer in proximity to said cells or cell-like particles; and (c) after passing through said guiding-layer, convert said wave to a detection signal; and
   wherein said change in mass is detected and/or investigated by means of using said surface acoustic wave device in delay-line configuration and detecting and/or investigating a phase-shift between said applied excitation signal and said detection signal.

In other aspects, the invention relates to **a use of a mass-sensitive chemical sensor** described above, and to **a kit** comprising such a mass-sensitive chemical sensor, for the detection and/or investigation of a change of mass caused by an interaction formed between an analyte ligand and a cell or cell-like particle.

### BRIEF DESCRIPTION OF DRAWINGS

FIGURE 1 depicts a schematic representation of cell adherence and their interaction with antibodies as analyte ligands. Selective antibodies (1) detecting different cell surface antigens interact with the cells (2) bound to the surface of the sensor chip (3). Inset: cells bind to E-Cadherin molecules immobilized on the chip surface by a dimerization process.

FIGURE 2 depicts the referenced phase-shift signals obtained upon adherence of intact native human tumour cells (MCF7) to the sensing surface and then upon interaction of three different antibodies (analyte ligands) that specifically recognise cell surface proteins (EMA, EP-CAM and E-Cadherin) to their respective protein targets presented on the surface of the intact native MCF7 cells.

FIGURE 3 depicts overlay plots of the time-course of referenced phase-shift following injections of antibodies binding to: (A) EMA; (B) Ep-CAM; and (C) E-Cadherin. Fitted-curves used to estimate binding constants are also shown.

### DETAILED DESCRIPTION

In a first aspect, the invention relates to **a method of detecting and/or investigating an interaction** between an analyte ligand and a cell or a cell-like particle, said method comprising the steps of:
- Providing a mass-sensitive chemical sensor having said cells or cell-like particles adhered to a sensing surface thereof, wherein the sensor is adapted to allow liquid to be introduced into the vicinity of the sensing surface; and
- Introducing a liquid comprising the analyte ligand into the vicinity of the sensing surface so as to allow interaction between analyte ligand and cells or cell-like particles; and
- Where an interaction is present, detecting and/or investigating the interaction by means of the change in mass at the sensing surface;
   wherein, said mass-sensitive chemical sensor comprises a surface acoustic wave device adapted to: (a) stimulate a shear-horizontal surface acoustic wave (SH-SAW) upon the application of an excitation signal; (b) propagate said wave through a guiding-layer in proximity to said cells or cell-like particles; and (c) after passing through said guiding-layer, convert said wave to a detection signal; and
   wherein said change in mass is detected and/or investigated by means of using said surface acoustic wave device in delay-line configuration and detecting and/or investigating a phase-shift between said applied excitation signal and said detection signal.

Terms as set forth hereinafter are generally to be understood by their common meaning unless indicated otherwise.

Where the term "comprising" or "comprising of" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group which preferably consists of all and/or only of these embodiments.

In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and preferably ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect.

Where an indefinite or definite article is used when referring to a singular noun, e.g. "a", "an" or "the", this includes a plural of that noun unless something else is specifically stated.

As used herein, an "analyte ligand" shall refer to a chemical, biological, or other, moiety or entity able to interact, directly or indirectly (preferably, directly), with the cell or cell like particle adhered to a sensing surface of a mass-sensitive chemical sensor essentially as described herein, and the presence, absence, amount of, or likelihood thereof, which is to be assessed qualitatively or quantitatively. Exemplary analyte ligands include, for example, without limitation: (i) synthetic or natural components such as small molecules, nucleic acids, peptides, aptamers, proteins and protein complexes, e.g. antibodies, antibody fragments or variants; (ii) replicating organisms such as viruses, cells, such as, for example, eukaryotic and/or prokaryotic and/or bacterial cells: and (iii) biologically/metabolically-relevant components such as toxins, hormones, cytokines, drugs or drug candidates, carbohydrates, glycoproteins, and lipids. Other specific classes of analyte ligands having particular biological effects on eukaryotic cells include infectious viral particles, invasive bacteria, other eukaryotic cells such as immunomodulatory cells or eukaryotic parasites and cholesterol and/or fat particles.

As used herein, an "interaction" between a cell or cell like particle and an analyte ligand shall refer to, for example, without limitation, a compositional, chemical, biological, or physical change in the overall composition, structure and/or state of the cell or cell like particle which, under the conditions provided, occurs predominantly or exclusively upon the cell or cell like particle coming into contact with the respective analyte ligand. For example, the interaction increases the mass of the cell due to formation of a combined cell/analyte-ligand composition. Preferably, the change in the overall composition, structure and/or state of the cell or cell like particle is proportional to the amount of analyte ligand in contact with the cell. In this context, "contact" may refer to spatial proximity, i.e. where atoms forming part of the analyte ligand move sufficiently close to atoms forming part of the cell or cell like particle that chemical bonds, e.g. hydrogen bonds, covalent bonds, pi-bonds, chelation bonds, etc, may be formed. Alternatively, "contact" may refer to a change in the environment of the cell or cell-like particle such as brought about by the presence of the analyte ligand or other factor, solute or liquid.

In certain embodiments of the present invention, the analyte ligand interacts but does not penetrate the surface of the cell or cell-like particle. For example, in certain of such embodiments the analyte ligand is not an antibacterial ligand. In other certain embodiments, the interaction of the analyte ligand with a cell or cell-like particle does not cause a change in the viscoelastic properties of said cell or cell-like particle. For example, the analyte ligand does not cause destabilisation of a lipid bilayer (such as a membrane) that comprises the cell or cell-like particle. In yet other embodiments of the present invention, the interaction of the analyte ligand does not lead to the formation of pores in the outer compartment (such as an outer membrane) of the cell or cell-like particle. For example, in such embodiments, upon interaction of said analyte ligand to said cell or cell-like particle, the cell or cell-like particle (preferably a cell) remains intact or substantially intact; that is, any penetration or structural changes upon interaction of the analyte ligand do not lead to gross conformational or other changes in the cell or cell-like particle, as such changes can be detected by, for example, amplitude of a detected signal from a SH-SAW sensor.

Acoustic wave sensor technology has shown broad application in detecting materials in various fields. Acoustic wave sensors detect materials by generating and observing an acoustic wave. As the acoustic wave propagates through or on the surface of the material, any changes to the characteristics of the propagation path affect the velocity and/or amplitude of the wave. The amplitude, frequency, and/or phase characteristics of the sensor can be measured and correlated to a corresponding physical quantity.

Several different types of acoustic wave devices have been developed, and while generally all acoustic wave sensors will function in gaseous or vacuum environments, all have only limited success in measuring water soluble or biological samples, especially when in contact with viscoelastic cell layers. Bulk acoustic waves (BAW) propagate through a medium. The most commonly used BAW devices are the thickness shear mode (TSM) resonator and the shear-horizontal acoustic plate mode (SH-APM) sensor, which are the commonly used waved-modes for QCM resonators. Conversely, waves that propagate on the surface of the substrate are known as surface waves. The most widely used surface wave devices are the surface acoustic wave sensor and the shear-horizontal surface acoustic wave (SH-SAW) sensor, also known as the surface transverse wave (STW) sensor.

Of the known acoustic sensors for liquid sensing, the Love wave sensor, a special class of the shear-horizontal SAW, has the highest sensitivity. To make a Love wave sensor, a dielectric waveguide coating is placed on a SH-SAW device such that the energy of the shear horizontal waves is focused in that coating. A chemo/biorecognition coating is then placed on the waveguide coating, forming the complete chemo/biosensor. SH-SAW and Love-wave SAW sensors are generally described, including their construction and application, in the two reviews cited herein.

Briefly, SH-SAW devices are constructed on a piezoelectric substrate material, and an electrical excitation signal is converted at interdigital transducers (IDT) into polarised transverse waves that travel parallel to the sensing surface, utilising the piezoelectric properties of the substrate material. In certain embodiments of the mass-sensitive chemical sensor, the adaptation to stimulate a SH-SAW, preferably a Love-wave, upon the application of an excitation signal is an IDT; such as one that has fingers aligned perpendicular to the wave propagation direction. In particular such embodiments, the fingers of the IDT are aligned parallel to the x-axis and wave propagation is in the z-axis. Piezoelectric substrate materials include quartz, LiTaO₃ and LiNbO₃. The quartz substrate may be cut in any suitable orientation to generate the desired wave. For example, AT-cut, ST-cut or Y-cut quartz Chrystal substrate may be used. Typically, IDT electrodes are made from one or more layers of gold and/or titanium (preferably gold) of between about 10 and 200nm thickness, and have between about 10 and 100 split-finger pairs with a wavelength (special periodicity) of between about 10 and 100um.

The acoustic wave thus generated is transmitted and is propagated confined to an independent guiding layer and not the substrate itself. Thus, the acoustic energy is concentrated within the guiding layer rather than in the bulk of the piezoelectric material. As the guiding layer is in proximity to the sensitive area of the sensing surface, chemical, biochemical, biological or physical events at such surface alter the properties of the acoustic wave as it is propagated within the guiding layer. The term "proximity" used in this context means that the wave propagated within the guiding layer can be affected by events (such as chemical, biochemical, biological or physical events) occurring at the sensing surface. Accordingly, in one embodiment, the guiding layer is considered to be in functional proximity to the sensing surface, such as the surface to which cells or cell-like particles can be adhered. In another embodiment, the guideline layer is in direct contact with the sensing surface, and in yet another embodiment, the guiding layer is within about 1 um, such as within about 500nm, 100nm, 50nm, 20nm or 10nm of the adhered cells. The guiding layer may be made by gold or SiO2 sputtered onto the piezoelectric substrate substrate.

A chemical sensor is created, when the coating of the SH-SAW sensing surface (in proximity to the guiding layer) binds specific chemical, biochemical or biological components, such as those comprised in liquids. SAW sensors may be operated on frequencies between about 10 MHz and 1 GHz, such between about 25 and 500 MHz or between about 75 and 250MHz. Based on the frequency, the penetration depth adjusts. With increasing frequency, the penetration depth decreases, and the sensitivity of SAW sensors increases by the square of the frequency; yielding a sensor highly sensitive for surface interactions. Before the disclosure of the present invention, it was understood that most events monitored are those that occur in close proximity to the sensing surface.

After passing through the guiding layer, the (modulated) acoustic wave is converted back into an electrical signal at another IDT, and such converted electrical signal serves as a detection signal. Typically, the second IDT has generally the same geometry as the first IDT

To read out the mass-sensitive chemical sensor used for the present invention, the SH-SAW is mounted as a delay-line together with a network analyser, or a reader unit is constructed for the electronic stimulation of the chips to generate and to compare the input and output electrical signals comprising a high-frequency generator, a I/Q demodulator and a frequency/phase comparator/analyser. Fig 2 of EP1577667 (and the corresponding description therein) describes a suitable arrangement to operate and analyse a SH-SAW sensing surface in delay-line mode and to detect, monitor and/or investigate a change in phase between the excitation signal applied to the first IDT compared to the (modulated) electrical signal generated at the second IDT after passing through the guiding layer and modulation by events at or near to the sensing surface. In particular embodiments, the SH-SAW sensor is operated in delay mode, and any phase-shift is detected indirectly using a change in frequency or velocity of the detection signal compared to the stimulation signal. In common to all such delay-line configurations, such "change" in phase, frequency or velocity of the detected signal is relative to the excitation signal, and not to the signal present before the occurrence of an event that modulated said signal, or not to a signal of a control sensing surface to which no (or a different) event occurred.

In certain embodiments, the mass-sensitive chemical sensor is not configured for, or operated in, resonance mode. For example, the first and second IDTs do not have an amplifier, such as a broadband amplified, connected between them, for example so as to form a feedback loop or resonating circuit.

The mass sensitivity of a SH-SAW sensor so configured depends on changes in the phase velocity inside the guiding layer (which can be affected by chemical, biochemical or physical events on the proximal sensing surface). In certain embodiments, the mass can be discriminated from viscoelastic effects by using both the phase shift and the amplitude shift of the surface acoustic wave.

A suitable SH-SAW sensing surface can be obtained from SAW Instruments GmbH (Bonn, Germany), and its specifications, characteristics and construction are generally as that described by Perpeet and coworkers (2006; Anal Lett, 39:1747-1757). Rocha-Gaso and coworkers (2009; Sensors, 9:5740-5769) describe an overview of many features and operational characterisitcs of other suitable SH-SAW sensing surfaces and how they may be used in a mass-sensitive chemical sensor.

The mass-sensitive chemical sensor may comprise an apparatus or device that has other components. For example, a sealed flow-cell may be used to introduce liquids over the surface of the sensing surface. The liquid can be introduced at a defined flow rate by a peristaltic or syringe pump, and the sample of cells, cell-like particles and/or analyte ligand can be injected into a buffer stream using either an injection valve for single injections, or an autosampler for rapid, standardised injections, enabling high throughput analyses. In addition, a microcontroller may processes the resulting signals (including recording and/or detection, investigation, monitoring or analysis of phase-shifts), and/or to control other aspects or features of the device or apparatus, for example via a computer which may be provided with corresponding software and an appropriate user interface.

The general arrangement of an SH-SAW sensor and how it may comprise part of an apparatus is schematically represented in Fig 1 of Gronewold (2007).

A suitable apparatus to use a suitable SH-SAW sensing surface is the sam®5 instrument which can be obtained from SAW Instruments GmbH (Bonn, Germany), and such apparatus includes applicable high-frequency electronic circuits and analysis systems to stimulate, detect and analyse phase-shifts upon events occurring on or in proximity to the sensing surface, as well as a flow-cell, syringe pump, autosampler and control computer and user interface.

In one embodiment of the present invention, said mass-sensitive chemical sensor is provided with, or forms part of, a flow cell. For example, said sensor is adapted by a flow-cell to allow liquids to be introduced into the vicinity of the sensing surface. In particular such embodiments, the design, orientation and/or size of the flow-cell may be adapted to facilitate such introduction in advantageous ways such as reduction of the volume of liquid comprising analyte liquid, or reduction of the likelihood of introduction or retention of bubbles or other obstacles in the vicinity of the sensing surface. The flow cell to be used in accordance with the present invention may be one which is preferably adapted for the determination of kinetic rate parameters of the studied interactions. The flow cell may be made of biologically compatible material, preferably selected from, but not limited to, polyoxymethylene, polymethylmethacrylate, polyvinyl chloride and injection-moldable thermoplastics, such as polystyrene or acrylonitrile-butadienestyrene. The dimensions of the flow cell may be suitable for determination of kinetic rate parameters for molecular interactions, i.e. in such embodiments the flow characteristics should allow for maintenance of the bulk solution concentration of the analyte ligand at, or very close to, the sensing surface. In certain embodiments, the flow-cell is one generally as, or preferably one specifically as, disclosed in WO2009/153189 copending EP10171508.4 or Community Designs 001789785-0001 to -0009.

It is a surprising effect of the present invention that a broad range of cell or cell-like particles can be used to detect and/or investigate the interaction thereto with an analyte ligand. In certain embodiments of the present invention, however, said cell or cell-like particle is larger than about two micrometer, or about five micrometer, in its largest dimension.

In a preferred such embodiment, said cell or cell-like particle is, in its largest dimension, larger then about 2 and smaller than about 50 µm, preferably between about 5 and about 25 µm, and more preferably between about 10 and about 20 µm. In other embodiments, said cell or cell-like particle is, in its largest dimension, between about 1 and about 200 µm, such as between about 1 and about 10 µm, or between about 5 and about 20 µm). In alternative embodiments, the cell or cell-like particle in its largest dimension is: between about 10 and 300 nm (such as a virus); is between about 0.5 to 5 µm (such as a bacterial); or is between about 4 and hundreds of micrometers 8such as up to about 300 µm), for example about 10 µm (such as a eukaryotic cell).

In another embodiment, the cell or cell-like particle is viscoelastic, especially a viscoelastic eukaryotic cell. The person of ordinary skill in the art will appreciate the meaning of "viscoelastic" in the context of cells and cell-like particles adhered to the surface of an acoustic sensor, and includes where interactions between such cells or cell-like particles and an analyte ligand can be detected by a mass-sensitive chemical sensor described and used as herein, but cannot be detected by a QCM-based sensor. Without being bound by theory, viscoelasticity of cells may damp and/or absorb the acoustic wave produced by QCM sensors, reducing the sensitivity of such sensors. For example, "viscoelastic" cells include those that are not rigid, such as cells that have been inactivated by methods analogous to those described in W02010/106331. Typically, intact cells - particularly intact eukaryotic (mammalian) cells - are viscoelastic and generate particular difficulties when adhered to the surface of acoustic sensors. Viscoelasticity of cells or cell-like particles may be determined, for example, by measuring the damping effect, loss of sensitivity, or change in amplitude upon their adherence to acoustic sensors such as QCM sensors or those described herein. Viscoelasticity of cells can be measured by a variety of methods, including those described by Peeters et al (2005; J Biocehm Eng, 127:237). TABLE 1, taken from Peeters (2004; "Biomechanics of single cells under compression", Thesis, Technical University of Eindhoven) describes parameters for the standard linear viscoelastic solid model (as geometrically represented in Figure 5.1 therein) as obtained for different cell types using several different experimental techniques and operating conditions. In certain embodiments of the present invention, the cell or cell-like particle has a viscoelasticity about that of a cell shown in TABLE 1 (and one or more of the associated parameters).

**TABLE 1**

| cell type | device | *F* or *P* | *T (°C')* | κ₁ (Pa) | κ₂ (Pa) | τ*ᵣ* (s) |
|---|---|---|---|---|---|---|
| chondrocytes | indentation | 50 nN | 25-37 | 1090 | 1140 | 1.32 |
| chondrocytes | micropipette | 100-500 Pa | 21 | 200 | 300 | 10 |
| hepatocytes | micropipette | 300 Pa | 24 | 87 | 33 | 0.18 |
| fibroblasts | microplates | 20-100 nN | 23 | 960 | 840 | 13 |
| endothelial cells | micropipette | 200 Pa | 23 | 100 | 200 | 39.5 |
| neutrophils | micropipette | 40 Pa | 22 | 31 | 76 | 0.22 |
| neutrophils | micropipette | 20 Pa | 22 | 28 | 74 | 0.18 |
| myoblasts | compression | 0.1-1 µN | 37 | 2120 | 1960 | 0.30 |

| | | | | | | |
|---|---|---|---|---|---|---|
| *F* = applied force, *P* = applied pressure, *T* = temperature. Non-linear viscoelastic model used to describe the dynamic experiments with spring constants κ₁, κ₂(ε_{z},) and relaxation time τ*ᵣ*. | | | | | | |

In a certain preferred embodiment of the present invention, said cell or cell-like particle is a cell, such as a eukaryotic cell. In particular such embodiments, said eukaryotic cell is one selected from the list comprising of: a mammalian cell, a yeast cell, an insect cell, and a plant cell. Yet more preferably, said eukaryotic cell is a mammalian cell, and most preferably it is a human cell.

In another preferred embodiment of the present invention, said cell is an intact, whole, live, vital, potent, native and/or active cell. In a further embodiment, the nucleus and/or outer-membrane of said cell is intact. In another related embodiment, said cell is essentially unaltered as compared to its composition, structure and/or state before adherence to said sensing surface was effected. In yet another related embodiment, said cell is metabolically active, or is a cell that has not had its normal biochemistry substantially altered or arrested. In alternative such embodiments, said cell is a prokaryotic and/or bacterial cell.

In other certain preferred embodiments of the present invention, the cells or cell like particles of the instant invention are not rigid, and/or, if cells, have not been inactivated, fixed, and/or crosslinked, e.g. by application of crosslinking agents and/or organic solvents, e.g. formaldehyde. In one embodiment, said cell or cell like particle is not a prokaryotic and/or bacterial cell (for example not an E.coli or Legionella bacterial cell). In yet another embodiment, the analyte is not a metal ion, such as a heavy metal ion, e.g. a cadmium or mercury ion.

In an alternative certain embodiment of the present invention, said cell or cell-like particle is a cell-like particle, such as a nano- or microparticles (ranging in size from about 10nm about 200 µm, such as between about 1 to about 10 µm, or between about 5 and about 20 µm), such as those made from lipid layers, polymers or gels, for example a liposome. Other examples of cell-like particles include ghost cells (ie, cells without a nucleus), lipoproteins, vesicles and (flexible) polymer particles such as synthetic cells, for example the bed blood cell-mimicking synthetic biomaterial particles as described by Doshi and coworkers (2009, PNAS, 106:21495-21499). A cell-like particle of the present invention will resemble, as the term implies, a cell, particularly with respect to those properties relevant to investigation on acoustic sensors, namely size and/or rigidity/viscoelasticity. Hence, a cell-like particle as contemplated by the present invention may have a size and viscoelastic behaviour similar to a cell, for example, a eukaryotic cell, e.g. a human cell. In certain embodiments, the cell-like particle is a ghost cell, a synthetic cell or other (flexible) polymer particles. In alterative embodiments, the cell-like particle is not a vesicle or a liposome. In yet other embodiments of the present invention, the cell-like particle is not a synthetic (lipid-based) vesicle or liposome, for example, it is not a vesicle or liposome formed by solvation and evaporation and/or sonication of lipids in and from organic solvents. In certain embodiments of the invention, a (lipid-based) vesicle or liposome cell-like particle is one that has been isolated from a natural system. In yet other certain embodiments, the (lipid-based) vesicle or liposome cell-like particle may be a natural (or synthetic) such cell-like particle that encloses a medium that is substantially different to the outer medium. For example, the enclosed medium may comprise active moieties such as chemicals, drugs or drug candidates, or may include organic solvents and/or colloidal components such as non-particles or precipitates.

In one embodiment, said mass-sensitive chemical sensor does not comprise a hydrogel located on the surface of said sensor. The term "hydrogel" refers to a colloid gel in which particles are dispersed in water and cross-linked. In a preferred embodiment, said mass-sensitive chemical sensor having said cells or cell-like particles adhered to a sensing surface thereof was not prepared by hydrolising a hydrogel comprising said cells or cell like particles. In another embodiment, said mass-sensitive chemical sensor does not comprise a polyelectrolyte located on the surface of said sensor, such as does not comprise polyallylamine hydrochloride (PAH) or polysodium 4-styrenesulphonate (PSS). In yet another embodiment, said method is not practiced for the detection and/or quantification of the amount of bacteria present in a sample.

In one certain preferred embodiment of the present invention, the mass-sensitive chemical sensor, that has cells or cell-like particles adhering to a sensing surface thereof, is provided by exposing cells or cell-like particles to a sensing surface adapted to capture cells or cell-like particles so as to adhere said cells or cell-like particles to said sensing surface, wherein said exposure of said cells or cell-like particles is conducted before (preferably directly before) the step of introducing the analyte ligand into the vicinity of the sensing surface. In a particular such embodiment, the cells or cell-like particles are exposed to said sensing surface present within a device used to detect and/or investigate said interaction.

Accordingly, in a certain preferred embodiment of the method of the present invention, the first "providing" step is fulfilled by steps comprising at least: (i) providing a mass-sensitive chemical sensor adapted to adhere cells or or cell-like particles wherein the sensor is adapted to allow liquid to be introduced into the vicinity of the sensing surface; and (ii) introducing a liquid comprising cells or cell-like particles into the vicinity of the sensing surface so as to allow adherence of said cells or cell-like particles to said sensing surface. In a particular such embodiment, the introducing step for cells or cell-like particles is conducted substantially similarly to the introducing step for liquid comprising the analyte ligand, such as within the same apparatus or device and/or without physical manipulation or insertion/removal of the sensing surface having said cells or cell-like particles adhered thereto.

Where it is referred to herein to said exposure of said cells or cell-like particles being conducted "directly" before the step of introducing the analyte ligand into the vicinity of the sensing surface, it shall be understood to mean that no material intermediate process or steps are performed in between (a) the step of exposing the cells or cell-like particles to the adapted sensing surface, and (b) introducing a liquid comprising the analyte ligand into the vicinity of the sensing surface. For example, there may not be an additional treatment, inspection, fixation, or inactivation step, or the like. In general terms, "material" intermediate steps that, in such embodiments, do not form steps of the present inventive method include: (1) Growing the cells on a sensor surface, such as for a period of time between 1 and 24 hours; (2) Fixing cells on the sensor surface, such as by treatment with formaldehyde; (3) Microscopic visualization of cells on the sensing surface; (4) Pretreatment of the sensing surface, before being mounted into a device; and/or (5) Stabilisation of the sensing surface once in the device, such as for between several hours or overnight. Specific examples of intermediate steps that, in such embodiments, do not comprise a step of the method of the present invention, include: maintaining the cells at reduced temperature (e.g. 2-8 degC), snap freezing, heat treatment under reduced pressure, toxin treatment, organic solvent treatment, and chemical cross-linking. Organic solvent treatment typically removes lipids and water from the cells and precipitates cellular proteins, whereas cross-linking reagents form intermolecular bridges between surface components of cells. Organic solvent treatments include the use of acetone, methanol and/or ethanol, typically at approximately -20 degC. Cross-linking approaches are those employing, for example, formalin, formaldehyde or paraformaldehyde, optionally in conjunction with a surfactant and/or methanol. Other examples include the fixation protocols described by Brock et al. (Cytometry 1999, 35: 353). However, an intervening pause (for example, for up to about 5 hours), such as for equilibration, or, e.g., a wash step, after adherence of the cells and before introduction of the liquid comprising analyte ligand, shall be considered within the bounds of the present invention.

As set out in WO2010/106331, for example, on pages 6, bottom para., to page 8, second para., and page 8, bottom para., to page 9, top para., and as shown in the examples therein, the methods of this prior art necessarily include such cumbersome and time consuming material intermediate processes or steps. By virtue of, among other advantages, the elimination of any one (or more) of these requirements, the instant invention provides for methods, uses and kits that are not only much easier to apply, but can also be considered as considerably more versatile and cost-effective. In addition, the results obtained when employing the methods, uses and kits according to the present invention are more accurate, as they detect interactions between components that are present in a state more close to their native state. Hence such data can be considered as more valuable, and may provide a deeper understanding of the physical, chemical and/or biological processes under scrutiny.

More particularly, using the protocols and routines of the present invention, it is possible to detect and/or investigate an interaction between a cell or cell-like particle and an analyte ligand and provide one or more alternatives or improvements over the prior art. By way of non-limiting example, the present invention may provide one or more of the following specific alternatives or improvements over the prior art, and/or other alternatives or improvements that become apparent when compared against prior art method:
- all important steps of the method, for example from adhering the cells to the sensing surface and including the regeneration of the sensing surface by their removal, to a reusable state, can potentially be performed without removal of the sensor from the flow cell and/or from the apparatus in which it is mounted;
- aggressive or hazardous chemicals (e.g. formaldehyde, paraformaldehyde), or harsh environmental conditions (e.g. -20 °C), need not be applied, such as to fix of otherwise inactivate cells in order to provide sufficient rigidity for sensitive detection. They may, however, be used to clean or sterilize the apparatus between experiments;
- the lower overall number of required steps (and/or the duration of certain individual steps) greatly reduces the time which needs to be allotted for a given experiment (typically a few minutes to a few hours vs. typically one to a few days for known methods);
- there are fewer limitations as to the selection of the analyte ligand and/or the cell or cell-like particle, including less limitations with respect to the viscoelastic behaviour of the cell or cell-like particle, nor less limitations with respect to those cells able to be grown on a sensor surface; in particular the present invention may be adapted to perform with, e.g. eukaryotic cells, bacteria, nanoparticles, and the like;
- the same apparatus and/or sensor may be used for essentially a wide range of cells and cell-like particles, such as those laid out above, with only routine adaptations known to the person of ordinary skill. Thus providing substantial savings in equipment costs, training time, personnel costs and set-up time;
- the data obtained come from cells and cell-like particles in a state that more closely mimics or otherwise represent their natural or naïve state, and are, hence, more scientifically meaningful and easier to interpret; and/or
- the sensor may be more easily regenerated and reused, including for control experiments, where the control experiment potentially more closely resembles the original experiment as compared to in the methods described in the art. Hence, time and money may be saved, as well as results further improved.

The inventors made the surprising invention that by use of the specific type of sensor of the invention in a specific way and by monitoring a specific change in wave property (in each case as described herein), are provided simple, flexible, cost effective and otherwise alternative or improved methods that are amenable to such a broad range of applications in the study of interactions between an analyte ligand and a cell or cell-like particle.

In a particular embodiment of the present invention, said sensing surface, prior to the adhering of said cells or cell-like particles, is adapted to comprise a binding moiety that is capable of binding to said cells. In a preferred such embodiment, said binding moiety is selected from the group comprising of: polymers/polymeric surfaces, charged or uncharged hydrophilic surfaces, antibody (or antibody variant or derivative), protein, nucleic acid, carbohydrate, glycoproteins, lipid, protein complex or aptamer. In a particularly preferred embodiment, said binding moiety is selected from the group comprising of: cadherins or antibodies.

In another particular embodiment of the present invention, said sensing surface comprises a gold, titanium or a silicon surface. In a preferred such embodiment, said gold surface is functionalised with -COOH, or said silicon surface is functionalised with organic silanes. In other embodiments, the surface comprises gold nano-particles or gold nano-posts (Kandulski et al, 2003; Acta Physica Polonica A, 104:495-502), and/or the surface comprises a polymer (such as a hydrogel) to extend the 3-dimentional surface to which binding moieties may be attached. These, and other, modifications to the sensing surface can be used to increase sensitivity and/or reduce non-specific binding of analyte ligand to the surface.

The person of ordinary knowledge and skill will now, upon disclosure of the present invention, readily determine suitable surfaces (and their functionalisation) for the sensing surface, the binding moiety and how to bind them to such surface, and the nature, concentration of, and method to be used to adhere the desired cells or cell-like particles to such sensing surface in order to practice the method of the present invention. For example, application notes obtainable from SAW Instruments GmbH, or publications described in the reviews cited above, provide numerous examples and methodologies to prepare SAW sensing surfaces with suitable binding moieties to adhere a broad range of cells or cell-like particles for which the person of ordinary skill may wish to utilise in the present invention. The appropriate choice of surface and binding moiety may, for example, depend on the specificity of adherence that is desired (for example, using antibody binding moieties if cell-specific adherence is desired, or carbohydrate binding moieties if non-specific adherence via to cell-surface glycoproteins is desired), or if regeneration/reuse of the sensing surface by removal of adhered cells is desired. By way of non-limiting example, some specific combinations are given in TABLE 2 below.

**TABLE 2**

| **Surface/ functionalisation** | **Binding moiety** | **Cell or cell-like particle** | **Citation** |
|---|---|---|---|
| Gold/-COOH | Antibody | Lymphocytes | Suraniti et al, 2007; Lab Chip, 7:1206-1208 |
| Gold/-COOH | 1) VCAM-1 = Vascular cell-Adhesion Molecule 1 2) Thio-Glucose; Heparin | MV3 cells / Membrane preparations thereof | Schlesinger et al, 209; Thromb Haemostat 102:1-7 |
| Gold/-COOH | Antibody | E.coli, other bacteria and virus | Moll et al, 2008; ITBM-RBM 29:155-161 |
| Titanium / Aminosilanized | Amino | E.coli | Pyuy et al, 1998; Biosens Bioelectron 13:839-845 |

The present invention can be used to detect and/or investigate the interaction between a cell or cell-like particle with a broad range of different analyte ligands. Accordingly, in certain particular embodiments of the present invention, the analyte ligand is selected from the group consisting of: antibody, protein, nucleic acid, cell, peptide, drug or drug candidate, carbohydrate and lipid, or is one selected from those defined or otherwise described elsewhere herein. In preferred embodiments, the analyte ligand is a protein, such as an antibody (or a variant or derivative thereof).

In a particular embodiment of the present invention, the detection/investigation of the mass-change is effected by the use of two excitation signals applied to the sensing surface, differing in frequency, such as two frequencies that differ to give a difference in phase of about 180°, and said change in mass is detected by a method that comprises the detection of two of said phase-shifts, one phase-shift between each pair of said applied excitation signals and its corresponding detection signal. In particular embodiments, the frequency range used is between about 130 and 170MHz, with the two frequencies differing by about 0.3MHz to give a difference in phase of these two frequencies of about 180°. Perpeet and coworkers (2006) and EP1577667 describes the two-frequency method, how to practice and analyse a SAW sensor using two-frequencies and the advantages it brings.

The commercially available sam®5 instrument from SAW Instruments GmbH (Bonn, Germany) utilises such detection methodology. Accordingly, in a preferred embodiment, said two phase-shifts are analysed by using a method substantially as described in EP1577667, defined herein as a "two-frequency method", wherein said method is a method for determining the mass of an analyte, which analyte is deposited onto a surface of a surface wave sensor in contact with a liquid comprising said analyte, wherein the sensor comprises an emitting electrode structure which excites a vibration on said surface, and wherein the sensor comprises a receiving electrode structure which detects a modified vibration on said surface, wherein the difference between the original and the modified vibration is registered and converted to an output signal, wherein the mass of said analyte that was deposited on said surface is inferred from said output signal, and wherein at least two different frequencies are excited and an output signal is recorded for each, and wherein the contribution to said output signal resulting from the dielectric properties of said fluid is inferred from the difference between the output signals, and wherein the output signal is corrected for said contribution, and wherein the deposited mass of said analyte is determined from the corrected output signal. Further specific embodiments of such method may be seen from the claims and/or description of EP1577667.

In other particular embodiments, any change in amplitude of the detected signal is detected, investigated and/or monitored as the analyte ligand is associated or dissociated from the cell or cell-like particle. Monitoring of such amplitude modulation can, in certain embodiments, enable the separation of signals resulting from mass changes from viscoelastic properties, as shown by Andrae and coworkers (2008) and Schlensog and coworkers (2004; Sens Actuators B, 101:308-315). Accordingly, in preferred such embodiments in respect of the analysis of the detected signal, the phase-shift is monitored (such as by using the two-frequency method) as well as amplitude modulation.

The sensitivity and real-time resolution of the method of the present invention provides the ability to qualitatively investigate the interactions detected. Accordingly, in preferred one embodiment, said method further comprises a step of: (x) monitoring the association of said analyte ligand from said cell or cell-like particle; and/or (y) monitoring the dissociation of said analyte ligand from said cell or cell-like particle. In a preferred such embodiment, said further step(s) includes a monitoring and/or recording of the rate of said association and/or dissociation. In a certain embodiment, said further step of monitoring the dissociation of said analyte ligand from said cell or cell-like particle is performed upon the introduction of a second liquid for dissociation of said interaction. The choice of such second liquid will depend on the nature and strength of the interaction to be dissociated. In yet another preferred embodiment, the association constant, dissociation constant and/or the affinity constant for the interaction between said analyte ligand and said cell or cell-like particle is estimated. In particularly preferred such embodiments, the method is practiced using an apparatus that automatically records (for example, by plotting or saving numerical values) the changes in phase-shift for the duration of the investigation of the interaction.

As may be seen within the non-limiting examples herein, the method may be used to study multiple interactions. Such studies may be done sequentially, and without dissociation of the first interaction, or may be consulted after dissociation of the first interaction. Accordingly, a certain embodiment of the present invention further comprises the step of detecting and/or investigating the interaction of a second analyte ligand to said cell or cell-like particle. In a preferred such embodiment, said step of detecting and/or investigating the interaction of a second analyte ligand to said cell or cell-like particle further comprises the introduction of a liquid comprising said second analyte ligand into the vicinity of the sensing surface prior to detecting the interaction of a second analyte ligand to said cell or cell-like particle. In other embodiment of the method, the second (or multiple) interactions may be detected and/or investigated in parallel to that of the first interaction. For example, the liquid comprising the analyte ligand into the vicinity of the sensing surface may comprise two or more different analyte ligands.

In alterative embodiments of the present invention, said second analyte ligand binds, preferably selectively, to the first analyte ligand. For example, in such embodiments the second analyte ligand may be a secondary antibody that selectively binds to the first antibody interacting with the cell or cell-like particle. Such secondary interactions, upon analysis of the resulting change in mass and/or amplitude modulation, can provide further information on the interaction of the first analyte ligand to the cell or cell-like particle. For example, information on the specificity of the interaction of the first analyte ligand to the cell or cell-like particle compared to its non-specific interaction to the sensing surface. In particular such embodiments, said second analyte ligand, such as a secondary antibody, is conjugated to a detectable-mass moiety. Such a detectable-mass moiety (for example a nano- or microparticle) can enhance or otherwise modify the change in phase (and/or amplitude) to aid the detection and/or investigation of the interaction of said second analyte ligand to said first analyte ligand.

In another advantage of the present invention, since they have not been chemically fixed or cross-linked to the sensing surface, the cells or cell-like particles can be relatively easily removed therefrom. Accordingly, in one embodiment, the present invention further comprises the step of removing the adhering cells or cell-like particles from said sensing surface. In a certain such embodiment, said removal is brought about by the introduction of a liquid for removal of said cells or cell-like particles into the vicinity of the sensing surface. By way of example, possible solutions for removing the cells or cell-like particles from the binding moieties include those that produce an acid or basic pH sift, comprise high-salt and/or EDTA. Upon removal of said cells or cell-like particles, the sensing surface (and hence the mass-sensitive chemical sensor) is regenerated. Accordingly, in a particular embodiment, said sensing surface is regenerated and/or reused. The advantages of regenerating or reusing the sensing surface include that not only are cost savings made, but it enables the conduct of certain control experiments.

Thereafter (or in addition to the first set of cells or cell-like particles), the sensing surface may be used to adhere to a second set of cells or cell-like particles. The person of ordinary skill will know if, and how to, if the "removed" sensing surface requires further treatments before it may be considered "regenerated", and this will depend on the nature of the binding moieties and functionalisation of the surface. Accordingly, in another one embodiment of the present inventions, said sensing surface is exposed to a second sample of cells or cell-like particles so as to adhere said cells or cell-like particles to said sensing surface.

In a second aspect, the present invention relates to **a method of quantifying, and/or estimating** the association, dissociation and/or affinity constant of an interaction between an analyte ligand with a cell or cell-like particle, said method comprising:
- Providing a mass-sensitive chemical sensor having said cells or cell-like particles adhered to a sensing surface thereof, wherein the sensor is adapted to allow liquid to be introduced into the vicinity of the sensing surface; and
- Introducing a liquid comprising the analyte ligand into the vicinity of the sensing surface so as to allow interaction between analyte ligand and cells or cell-like particles; and
- Where an interaction is present, detecting, investigating and/or monitoring the interaction by means of the change in mass at the sensing surface; and
- Monitoring the association of said analyte ligand from said cell; and/or
- Monitoring the dissociation of said analyte ligand from said cell; and
   - *preferably* computing an association, dissociation and/or affinity constant from results thereby obtained;
wherein, said mass-sensitive chemical sensor is one as defined or otherwise described herein; and
wherein said change in mass is detected, investigated and/or monitored as defined or otherwise described herein.

In a third aspect, the instant invention provides a **use of a mass-sensitive chemical sensor** to detect and/or investigate a change of mass caused by an interaction formed between an analyte ligand and a cell or a cell-like particle;
wherein, said mass-sensitive chemical sensor is one as defined or otherwise described herein; and
wherein said change in mass is detected and/or investigated as defined or otherwise described herein.

In a fourth aspect, the instant invention provides **a kit**, preferably a kit for the detection and/or investigation of a change of mass caused by an interaction formed between an analyte ligand and a cell or a cell-like particle, said kit comprising of:
- A mass-sensitive chemical sensor as defined or otherwise described herein, or an apparatus comprising or adapted to use said mass-sensitive chemical sensor; and
- Instructions to: (a) detect and/or investigate said change in mass as defined or otherwise described herein; and (b) practice a method as described or otherwise defined herein.

In certain preferred embodiments, said instructions are in printed form on paper. Said instructions may, for example, be added to a kit on a separate sheet of paper, or printed onto a container or package comprised of the kit. In certain other embodiments, said instructions are provided in electronic format, such as, for example, in the form of one or more electronic files stored on a magnetic or optic medium, such as a floppy disk, compact disc (CD), digital versatile disc (DVD) or USB/memory device. Alternatively, said electronic files are stored on one or more electronic file servers, and the kit provides a means for obtaining said stored files, such as an internet link or address, or an executable file which may establish a remote connection, and/or a log in code, by downloading said stored files from at least one of said servers.

It is to be understood that application of the teachings of the present invention to a specific problem or environment, and the inclusion of variations of the present invention or additional features thereto, will be within the capabilities of one having ordinary skill in the art in light of the teachings contained herein. Representative examples of the methods, uses, kits and other aspects of the present invention should not be taken to limit the scope of the present invention to only such representative examples.

All references, patents, and publications cited herein are hereby incorporated by reference in their entirety.

### EXAMPLES

EXAMPLE 1: Antibody binding to native whole eukaryotic cells. The inventors made the surprising invention that by using in a particular way a specific type of acoustic wave-based mass-sensitive chemical detector, and by detecting a change in a particular property of an acoustic wave stimulated on the surface of such detector, they could detect and investigate antibody interaction to extra-cellular domains of membrane proteins presented on intact mammalian cells.

Cell-surface proteins were recognised in their native environment; that is the cells were not inactivated or fixed, e.g. by applying organic solvents, with the consequential possibility of denaturation or other changes to the constitution, abundance or even presence of such cell-surface proteins. The antibodies bound such protein targets presented by the cells according to both abundance and affinity of the protein. Using the phase signal, binding measurements were performed using the sensing surface of a SAW-based mass-sensitive chemical detector, despite the presence of a challenging biological matrix consisting of the highly viscoelastic layer of native cells adhering to the surface of the sensor.

In this example, whole breast cancer cells are adhered to the surface of a SAW sensor chip by dimerization with E-Cadherin monomers. Cadherins are Ca²⁺-dependent transmembrane adhesion proteins forming and stabilizing cell contacts in different tissues. The change in mass brought about by interaction between specific antibodies, and surface proteins (with extracellular domains) embedded in the plasma membrane of the whole intact cell was detected as a phase-shift (see FIGURE 1). This is an application which is not possible using SPR or QCM detectors. Using such surface acoustic wave sensors in this way, it is simple to perform this quantitative assay and it is a valuable addition to the armoury for researchers who are investigating antibodies against or targeting towards membrane receptors, particularly those of intact eukaryotic cells.

Firstly, the adherence of intact human tumour cells (MCF7) via dimerization to the E-cadherin monomers presented on the SAW-sensing surface could be detected after each of two sequential injections of running buffer comprising MCF7 cells. Detection was made by a change in phase of the signal at the detector IDT after it had passed along the sensing surface (first two increases show in FIGURE 2). Second, and surprisingly, the change of mass (as detected by a corresponding change in phase) resulting from the interaction of three sequential injected antibodies with their corresponding cell-surface protein targets presented on the surface of the intact MCF7 cells. Indeed and yet further surprisingly, the detected effect on phase-shift quantitatively correlated to the quantity and distribution of the three different cell surface protein targets presented by these cells (FIGURE 2): (1) EMA is a heterogenous group of heavily glycosylated proteins expressed by a wide range of tumours; (2) Ep-CAM is a type I membrane protein frequently expressed by epithelial cells and many carcinomas. Due to its high abundance, it showed highest response at antibody injection; (3) E-Cadherin, the adhesion proteins. Extremely low buffer effect was observed upon injection of cells or of the glycerol-containing antibody solution. Antibody binding was controlled by repeating the experiment with injections of the antibody but to SAW-sensors that had not previously been exposed to MCF7 cells (data not shown). One unused channel of the SAW-sensor was used as a phase-reference to reference the phase-shifts collected.

Secondly, Origin software (OriginLab Corporation) was used to evaluate kinetic data of association and dissociation of each antibody to its respective protein target (FIGURE 3). Fits based on a 1:1 binding model were applied, and kinetic data evaluated as far as possible since exact original antibody concentrations were unknown, and estimates of certain rate constants are shown in TABLE 3below.

**TABLE 3**

| **Antibody** | **Kobs** | **Kdiss** | **Aeq** |
|---|---|---|---|
| anti-EMA | 0.0610 | 0.0026 | 0.2769 |
| anti-Ep-Cam | 0.0078 | 0.0014 | 3.3457 |
| anti-E-cadherin | 0.0374 | 0.0051 | 0.4190 |

Thirdly, the MCF7 cells were removed from the sensing surface, and the chip surface accordingly regenerated, by injecting buffer supplied with 500mM EDTA (data not shown).

The detection and measurements of the interaction between the (MCF7) cells and (antibody) analyte ligands were performed with a standard sam®5 biosensor (SAW Instruments, Bonn Germany) using surface acoustic waves, equipped with SH-SAW gold chips functionalised with -COOH stable self-assembled monolayers (SAM) (Schreiber, 2000; Prog Surf Sci 65: 151-256; Gronewold et al, 2009; J Prot Res 8: 3568-3577). A rhE-Cadherin/Fc Chimera (recombinant human, R&D Systems, #648-EC ) was bound via carbodiimide chemistry in PBS plus EDTA (500mM) running buffer (conserving the monomers at immobilization) to the functionalised SAW sensor chip at a loading density of about 2.5ng/cm² (about 28fmol/cm²), using the method generally as described in Schlensog (2004). Running buffer was then exchanged to PBS plus 1.3mM CaCl₂ plus 1mM MgSO₄to enable E-cadherin dimerization for capture and adherence of injected MCF-7 cancer cells (at 10⁶ cells/mL in Running buffer) with the chip present in the same apparatus. Without any material intervening steps or manipulation of the chip, interaction of three different protein targets (presented on the surface of the MCF7 cells) with their respective specific antibodies was then monitored upon injection of the corresponding antibody (anti-human E-Cadherin and anti-human EMA antibodies from Thermo Scientific Pierce; Anti-human Ep-CAM antibody from BioLegend) at a dilution of 1:100. The three protein targets investigated were: (A) EMA - Epithelial Membrane Antigen - a 265-400kDa transmembrane protein; (B) Ep-CAM ( also known as EPG40) - epithelial Cell Adhesion Molecule - a 40kDa transmembrane epithelial glycoprotein; and (C) E-Cadherin, a 87.7kDa cadherin protein presented by epithelial cells (note, dimerization between surface-bound E-Cadherin molecules and those displayed on the surface of the cells is also used in this experiment to adhere the cells to the sensing surface).

EXAMPLE 2: Detection of binding analyte ligand to cell-like particles. The inventors demonstrate the further applicability of their inventive method by detecting the interaction of thrombin to the surface of anti-thrombin antibody-coated microparticles. Murine anti-human thrombin antibody (Haemtech) is bound to magnetic (cell-like) microparticles (Dynal) according to manufacturer's instructions. The cell-like particles are adhered to a SH-SAW sensing surface (as described above) by placing a magnet under the surface. In other experiments, they are adhered to a SH-SAW sensing surface that comprises goat anti-mouse-antibody antibody (Sigma) as binding moiety. Running buffer comprising human thrombin (Haemtech) is introduced into the vicinity of the sensing surface with adhered cell-like particles, and interaction of the thrombin to the cell-like particle using a shift of phase from a delay-line-configuration of the sensor is detected and/or investigated substantially as described above.

EXAMPLE 3: Antibody binding to native whole prokaryotic cells. The inventors further demonstrate the applicability of their inventive method by detecting antibody interaction to intact prokaryotic cells. In this example, the SH-SAW sensing surface as described above comprises antibodies that bind pathogenic E. coli species. Interaction of analyte ligands being antibodies against: (1) P1 / F1 pilus adhesion; (2) aerobactin receptor protein; (3) lipopolysaccharids; and/or (4) IroN siderophore receptor; are monitored using a shift of phase from a delay-line-configuration of the sensor, substantially as described above, after introduction of liquid comprising said antibody/antibodies into the vicinity of the sensing surface with adhered prokaryotic cells.

EXAMPLE 4: Kit of parts. A package is prepared that contains: (1) a standard sam®5 biosensor gold chip functionalised with -COOH stable self-assembled monolayers (SAW Instruments, Bonn Germany); and (2) a printed sheet describing the steps and reagents required to detect and/or investigate an interaction between an analyte ligand and a whole mammalian cell, by using a sam®5 biosensor instrument (SAW Instruments, Bonn Germany). Other kits are prepared that contain such instructions on a CD, and that contain one or more reagent (such as a binding-moiety) useful to practice such method. In another kit includes: (i) a sam®5 biosensor instrument (an instrument suitable and adapted for use with a standard sam®5 biosensor gold chip functionalised with -COOH stable self-assembled monolayers); and (iii) an instruction manual comprising a printed sheet of instructions as described above.

## Claims

1. **A method of detecting and/or investigating an interaction** between an analyte ligand and a cell or a cell-like particle, said method comprising:
• Providing a mass-sensitive chemical sensor having said cells or cell-like particles adhered to a sensing surface thereof wherein the sensor is adapted to allow liquid to be introduced into the vicinity of the sensing surface; and
• Introducing a liquid comprising the analyte ligand into the vicinity of the sensing surface so as to allow interaction between analyte ligand and cells or cell-like particles; and
• Where an interaction is present, detecting and/or investigating the interaction by means of the change in mass at the sensing surface;
wherein, said mass-sensitive chemical sensor comprises a surface acoustic wave device adapted to: (a) stimulate a shear-horizontal surface acoustic wave (SH-SAW) upon the application of an excitation signal; (b) propagate said wave through a guiding-layer in proximity to said cells or cell-like particles; and (c) after passing through said guiding-layer, convert said wave to a detection signal; and
wherein said change in mass is detected and/or investigated by means of using said surface acoustic wave device in delay-line configuration and detecting and/or investigating a phase-shift between said applied excitation signal and said detection signal.

2. The method of claim 1, wherein said cells or cell-like particles are:
• Larger than about two micrometer in their largest dimension; and/or
• Viscoelastic.

3. The method of claim 1 or 2, to detect and/or investigate an interaction between an analyte ligand and a cell; and
- *preferably* wherein said cell is a eukaryotic cell;
- *preferably* wherein said cell is selected from the list comprising of: mammalian, yeast, insect and plant; and/or
- *preferably* wherein said cell is mammalian.

4. The method of claim 3, wherein:
• said cell is intact, whole, live, native, vital, potent and/or active; and/or
• the nucleus and/or the outer-membrane of said cell is intact.

5. The method of any one of claims 1 to 4, wherein said mass-sensitive chemical sensor having said cells or cell-like particles adhered to a sensing surface thereof is provided by exposing said cells or cell-like particles to a sensing surface adapted to capture cells so as to adhere said cells or cell-like particles to said sensing surface; and
wherein said exposure of said cells or cell-like particles is conducted directly before the step of introducing the analyte ligand into the vicinity of the sensing surface.

6. The method of any one of claims 1 to 5, wherein said sensing surface, prior to adhering of said cells or cell-like particles, is adapted to comprise a binding moiety that is capable of binding to said cells or cell-like particles; and
- *preferably* wherein said binding moiety is selected from the group comprising of: antibody, protein, nucleic acid, lipid, glycoprotein and carbohydrate; and/or
- *preferably* wherein said binding moiety is selected from the group comprising of: Cadherin and antibody.

7. The method of any one of claims 1 to 6, wherein said analyte ligand is selected from the group consisting of: protein, antibody (or antibody fragment or variant), nucleic acid, cell, peptide, drug or drug candidate, carbohydrate, lipid, and glycoprotein; and
- *preferably* wherein said analyte ligand is an antibody or an antibody fragment or variant.

8. The method of any one of claims 1 to 7, wherein two excitation signals are applied, differing in frequency, and where said change in mass is detected by a method that comprises the detection of two of said phase-shifts, one phase-shift between each pair of said applied excitation signals and its corresponding detection signal; and
- *preferably* wherein said two phase-shifts are analysed by using a two-frequency method.

9. The method of any one of claims 1 to 8, further comprising a step of:
• Monitoring the association of said analyte ligand from said cell or cell-like particles ; and/or
• Monitoring the dissociation of said analyte ligand from said cell or cell-like particles;
- *preferably* wherein the rate of said association and/or dissociation is monitored and/or recorded.

10. The method of claims 9, wherein the association constant, dissociation constant and/or the affinity constant for the interaction between said analyte ligand and said cell or cell-like particle is estimated.

11. The method of any one of claims 1 to 10, further comprising the step of detecting the interaction of a second analyte ligand to said cell or cell-like particle; and
- *preferably* by the introduction of a liquid comprising said second analyte ligand into the vicinity of the sensing surface.

12. The method of any one of claims 1 to 11, further the step of removing the adhering cells or cell-like particles from said sensing surface; and
- *preferably* by the introduction of a liquid for removal of said cells or cell-like particles into the vicinity of the sensing surface; and/or
- *preferably* wherein said sensing surface regenerated and/or reused.

13. The method of claim 12, wherein said sensing surface is exposed to a second sample of cells or cell-like particles so as to adhere said cells or cell-like particles to said sensing surface.

14. **Use of a mass-sensitive chemical sensor** to detect and/or investigate a change of mass caused by an interaction formed between an analyte ligand and a cell or a cell-like particle;
wherein, said mass-sensitive chemical sensor comprises a surface acoustic wave device adapted to: (a) stimulate a shear-horizontal surface acoustic wave (SH-SAW) upon the application of an excitation signal; (b) propagate said wave through a guiding-layer in proximity to said cells or cell-like particles; and (c) after passing through said guiding-layer, convert said wave to a detection signal; and
wherein said change in mass is detected and/or investigated by means of using said surface acoustic wave device in delay-line configuration and detecting and/or investigating a phase-shift between said applied excitation signal and said detection signal.

15. **A kit** comprising of:
• A mass-sensitive chemical sensor comprising a surface acoustic wave device adapted to: (a) stimulate a shear-horizontal surface acoustic wave (SH-SAW) upon the application of an excitation signal; (b) propagate said wave through a guiding-layer in proximity to said cells or cell-like particles; and (c) after passing through said guiding-layer, convert said wave to a detection signal, or an apparatus comprising or adapted to use said mass-sensitive chemical sensor; and
• Instructions to: (a) detect and/or investigate said change in mass by means of using said surface acoustic wave device in delay-line configuration and detecting and/or investigating a phase-shift between said applied excitation signal and said detection signal; and (b) practice the method of any one of claims 1 to 13 of the use of claim 14.
